# EUROPÄISCHE PATENTSCHRIFT

(11) **EP 0 991 381 B1**
(45) Veröffentlichungstag und Bekanntmachung des Hinweises auf die Patenterteilung: **04.06.2003**
(21) Anmeldenummer: 99910364.1
(22) Anmeldetag: 18.03.1999
(51) Int. Cl.: A61F 5/01

(54) **VORRICHTUNG ZUR STABILISIERUNG EINES GELENKS**
DEVICE FOR STABILIZING A JOINT
DISPOSITIF POUR STABILISER UNE ARTICULATION

(30) Priorität: 19.03.1998 DE 19811925
(43) Veröffentlichungstag der Anmeldung: 12.04.2000
(73) Patentinhaber: Albrecht GmbH, 83115 Neubeuern (DE)
(72) Erfinder: JAGODZINSKI, Michael, D-69123 Heidelberg (DE)
(74) Vertreter: Bauer, Friedrich
(86) Internationale Anmeldenummer: EP9901815
(87) Internationale Veröffentlichungsnummer: WO99047084

(56) Entgegenhaltungen:
- WO-A-96/16615
- DE-A- 19 631 632
- US-A- 4 856 500
- US-A- 5 685 830

## Beschreibung

Die Erfindung betrifft eine Vorrichtung zur Stabilisierung eines Gelenks, insbesondere eines Kniegelenks gemäß dem Oberbegriff des Anspruches 1.

Es sind Knieorthesen mit einer Oberschenkel- und Unterschenkelschiene bekannt, die jeweils über Kunststoffbänder mit Klettverschlüssen am Ober- bzw. Unterschenkel festlegbar sind und die zur Überbrückung des Kniegelenks mit einem Scharniergelenk verbunden sind. Das Scharniergelenk zwischen der Oberschenkel- und Unterschenkelschiene weist einen festen Drehpunkt auf, um den die Oberschenkel- und Unterschenkelschiene zueinander verschwenkbar sind. Über Metallstifte oder Kunststoffelemente kann die Schwenkbewegung auf Winkel zwischen 0° und 140° einschränkbar sein. Bei den bekannten Knieorthesen besteht der Nachteil, dass das menschliche Kniegelenk durch eine Knieorthese mit Scharniergelenk nur unvollkommen imitiert und stabilisiert werden kann. So hat sich in klinischen Studien gezeigt, dass bei den heute üblichen Knieorthesen lediglich eine Zugverminderung am vordere Kreuzband zu bewirken ist. Ferner führt die zirkuläre Fixierung der Ober- und Unterschenkelschienen an der Ober- bzw. Unterschenkelmuskulatur einerseits zu Durchblutungsstörungen, beispielsweise der Wadenmuskulatur, und andererseits zu nachteiliger Veränderung des Gangbildes.

Aus der EP 0 432 303 A1 ist eine Vorrichtung zur Stabilisierung eines Kniegelenks mit einer Oberschenkel-Fixierschiene und einer Unterschenkel-Fixierschiene bekannt, die über zwei seitliche Arme miteinander gelenkig verbunden sind. Diese beiden Fixierschienen sind über verstellbare Manschetten am Oberschenkel bzw. Unterschenkel festlegbar. Auf einer Seite dieser bekannten Vorrichtung ist ein Zugmittel vorgesehen, das bewirkt, dass mit zunehmender Streckung bzw. Beugung des Beines die Manschetten fester um den Oberschenkel bzw. Unterschenkel herum angezogen werden.

Im Zusammenhang mit Kreuzbandrissen besteht auch das Problem, dass die Zugentlastung der Kreuzbänder in Richtung des geschädigten Kreuzbandes in den Extrempositionen besonders groß sein muss, während die Zugentlastung in Richtung des gerissenen Kreuzbandes in der gebeugten Stellung geringer sein kann. Bei der Knieorthese gemäß der EP 0 432 303 A1 besteht der Nachteil, dass zwar die Orthese abhängig von der Position des Oberschenkels unterschiedlich stark am Bein festgelegt wird, jedoch keine differenzierte Stabilisierungskraft der Orthese vorgesehen ist, welche die Fehlfunktion des Kreuzbandes durch eine höhere Zugkraft in dessen Richtung, vor allem in den Extrempositionen des Beines, ersetzen kann.

Aus der WO96/16615 ist eine Manschette zur Stabilisierung eines Knies bekannt, die sich vom Oberschenkel über das Knie bis zum Unterschenkel erstreckt und insbesondere zur Stabilisierung des Kniescheibenbereiches dient. An beiden Seitenbereichen der Manschette ist jeweils eine Gelenkschiene mit zwei Schienenarmen befestigt, die gelenkig miteinander verbunden sind. Ein Zugmittel in der Form eines Kabels, das sich in einer gekreuzten Anordnung von einem Ende bis zum anderen Ende der Manschette erstreckt, drängt die Schienengelenke und damit die Manschette im Kniebereich nach vorne, so dass eine unerwünschte Rückwärtsverschiebung des Unterschenkels relativ zum Oberschenkel verhindert wird. Auch dort ist keine differenzierte Stabilisierungskraft der Orthese vorgesehen, welche die Fehlfunktion des Kreuzbandes durch eine höhere Zugkraft in dessen Richtung ersetzen kann.

Der Erfindung liegt die Aufgabe zugrunde, eine Vorrichtung zur Stabilisierung eines Gelenks der genannten Art anzugeben, die eine verbesserte Stabilisierung des Kniegelenks ermöglicht und dabei eine zusätzliche Zugverminderung an den Bändern des zu stabilisierenden Gelenks bewirkt.

Diese Aufgabe wird erfindungsgemäß durch die Merkmale des Anspruchs 1 gelöst. Danach verläuft das Zugmittel von der Vorderseite der ersten Fixierschiene beidseitig am Gelenk vorbei zu einem hintere Bereich der zweiten Fixierschiene, wobei es auf die erste Fixierschiene eine zum Gelenk und nach hinten gerichtete Zugkraft und auf die zweite Fixierschiene eine zum Gelenk und nach vorne gerichtete Zugkraft ausübt, so dass das erste Körperglied zum Gelenk und nach hinten und das zweite Körperglied zum Gelenk und nach vorne gespannt wird.

Erfindungsgemäß wurde erkannt, dass eine Gelenkstabilität nach einem Bänderriss, insbesondere einer Kreuzbandruptur, vorteilhaft durch ein Zugmittel stabilisierbar ist, das das vordere und/oder hintere Kreuzband außerhalb des Kniegelenks nachbildet. Ein besonderer Vorteil dabei ist, dass sich das Stabilisierungs- und Gangbild des Gelenks beim Patienten bei angelegter Knieorthese während der Flexion und Extension im Vergleich zum gesunden Kreuzband nahezu nicht ändert. Die am Oberschenkel festgelegte Oberschenkelschiene und die am Unterschenkel festgelegte Unterschenkelschiene sind durch das Zugmittel an den Seiten des Gelenks derart verbunden, daß das Zugmittel an den Fixierschienen dort gelagert ist, wo der extraartikulärprojizierte Isometriepunkt, beispielsweise des vorderen Kreuzbandes, angeordnet ist. Auf diese Weise ist das Zugmittel an der Außenseite des Kniegelenks angeordnet und weist einen den Kreuzbändern im Knie entsprechenden Verlauf, lediglich außerhalb des Kniegelenks nachgebildet, auf.

Die Knieorthese verhindert dabei insbesondere in den extensionsnahen Kniegelenkstellungen die vordere Instabilität, da dies der Hauptwirkung des durch das Zugmittel nachgebildeten vorderen Kreuzbandes entspricht. Die erfindungsgemäße Knieorthese wirkt dabei vor allem den Instabilitäten beim Gehen, insbesondere in der Extensionsphase des stabilisierten Kniegelenks entgegen. Der Längszug an einem Transplantat nimmt vor allem in der Extension des Kniegelenks zu, weshalb die Verwendung einer erfindungsgemäßen Knieorthese mit äußerlich durch das Zugmittel nachgebildeten Kreuzbändern als besonders vorteilhaft angesehen werden muß.

Ein ganz entscheidender Vorteil der erfindungsgemäßen Knieorthese liegt somit in der Anordnung des Zugmittels, das eine vom Beugezustand des Kniegelenks abhängige Zugkraft auf den Unterschenkel in Richtung zum Oberschenkelknochen ausübt. Bei fehlender Funktion des vorderen Kreuzbandes kann die Zugrichtung nach hinten auf die Schienbeinvorderkante gerichtet sein, wobei die maximale Zugwirkung bei 30° Beugung liegt. Bei fehlender Funktion des hinteren Kreuzbandes ist die Zugrichtung des seitlichen Zugmittels zwischen der Unterschenkel-Fixierschiene und der Oberschenkel-Fixierschiene so eingerichtet, daß das Zugmaximum bei 70° Beugestellung des Kniegelenks erreicht wird, wobei das Zugmittel in Seitenansicht in Richtung des hinteren Kreuzbandes seitlich am Knie angeordnet ist und ein Zugmaximum bei 70° Beugestellung des Kniegelenks hat.

Das Zugmittel ist bevorzugt als Seilzug oder flexibler Kunststoff- oder Drahtstab ausgebildet. Als Seilzug kann beispielsweise ein Kunststoffseilstück vorgesehen sein, das zu beiden Seiten des Kniegelenks angeordnet ist, oder es können auch mehrere Seilstücke separat vorgesehen sein, die die erste Fixierschiene mit der zweiten Fixierschiene an den Seiten des Gelenks verbindet. Statt eines Seilzugs kann aber auch ein flexibler Kunststoff- oder Drahtstab vorgesehen sein, der dann beispielsweise einen erhöhten Widerstand beim Auslenken der beiden Fixierschienen um eine Drehachse vorsieht. Das Zugmittel soll lediglich die Kreuzbänder extraartikulär nachbilden, und deswegen auch deren Eigenschaften in bezug auf die Aufnahme von Zugkräften optimal imitieren. Dabei kann auch vorgesehen sein, daß das Zugmittel in gewissem Rahmen elastische Eigenschaften besitzt.

Bei einer ersten Weiterbildung der Erfindung verläuft das Zugmittel von einer Vorderseite der ersten Fixierschiene, beispielsweise Unterschenkel-Fixierschiene, seitlich am Gelenk vorbei zu einem hinteren Bereich der Seitenfläche oder zur Rückseite der zweiten Fixierschiene, beispielsweise Oberschenkel-Fixierschiene. Es wird hierbei darauf hingewiesen, daß die Vorrichtung zur Stabilisierung eines Gelenks nicht auf Knieorthesen beschränkt sein soll. Die erfindungsgemäße Vorrichtung kann auch zur Stabilisierung sämtlicher anderer körpereigener Gelenke bei Lebewesen eingesetzt werden. Lediglich zur Vereinfachung wird die Vorrichtung zur Stabilisierung eines Gelenks am Beispiel einer Knieorthese dargestellt. Soweit in der folgenden Bschreibung von der Vorderseite des Gelenks gesprochen wird, handelt es sich dabei um die Seite, in der das Gelenk sperrt, so daß das Gelenk in der Vorderrichtung nicht weiter auslenkbar ist. Beim Kniegelenk ist die Vorderseite des Gelenks damit die in Gehrichtung vorn liegende Seite.

Im einfachsten Fall sind zwei Zugmittel vorgesehen, die jeweils an den Gelenkseiten verlaufen und die Oberschenkelschiene mit der Unterschenkelschiene verbinden. Bei der Gehbewegung wird das Knie dadurch stabilisiert, daß die Unterschenkelschiene unterhalb des Kniegelenks nach hinten gespannt wird, während die Oberschenkelschiene durch das Zugmittel und zugleich oberhalb des Kniegelenks nach vorne gespannt wird.

Bevorzugt ist das Zugmittel an der Seitenfläche der zweiten Fixierschiene, insbesondere der Oberschenkelschiene, über ein Umlenklager umlenkbar und verläuft von dort zur Vorderseite der zweiten Fixierschiene. Das Zugmittel ist dabei einerseits an der Vorderseite der Unterschenkelschiene festgelegt und andererseits an der Vorderseite der Oberschenkelschiene festgelegt. Infolgedessen bewirkt die Umlenkung einerseits, daß die Oberschenkelschiene zur Vorderseite des Oberschenkels hin gespannt wird und andererseits bewirkt die Festlegung vorne an der Oberschenkelschiene, daß das Zugmittel an der Vorderseite beispielsweise zum Verstellen oder zum Öffnen, wie es beispielsweise zum Anlegen der Vorrichtung notwendig ist, zugänglich ist.

Bei einer besonders bevorzugten Weiterbildung der erfindungsgemäßen Vorrichtung ist das Zugmittel als Seilring ausgebildet. Der Seilring besteht dabei aus einer Kunststoffkordel, die über einem Verschlußmittel, wie etwa einem Schnapp- oder Rastverschluß, zur Bildung des Seilrings verbindbar oder trennbar ist. Erfindungsgemäß kann der Seilring an der Vorderseite oben an der Unterschenkelschiene festgelegt sein und von dort zu beiden Seiten des Kniegelenks nach hinten verlaufen, um dort an der Umlenkstelle im Bereich der hinteren Seitenfläche der Oberschenkelschiene nach vorn umlenkbar zu sein. Von den seitlichen Umlenklagern an der Oberschenkelschiene verläuft der Seilring zur Vorderseite der Oberschenkelschiene, beispielsweise zu einem Seilringverschlußmittel. Das Seilringverschlußmittel läßt das Öffnen des Seilrings zu, um die Knieorthese an- bzw. abzulegen.

Bevorzugt ist ein erstes Stellelement zum Verlagern des Befestigungs- oder Umlenkpunktes des Zugmittels an der Vorderseite der ersten Fixierschiene, d.h. der Unterschenkelschiene, vorgesehen. Das Verstellmittel kann beispielsweise verschiedene rasterartig angeordnete Befestigungspunkte für einen Seilring aufweisen, so daß dieser in verschiedenen Befestigungspositionen einhängbar und anschließend in eine Richtung weg vom Kniegelenk spannbar ist. Es kann aber auch jede andere bekannte Spannvorrichtung einsetzbar sein, um eine optimale Positionierung des Seilzugs gegenüber der ersten Fixierschiene zu ermöglichen.

Über ein Seilverkürzungsmittel kann zusätzlich die Länge des Zugmittels einstellbar sein. Bevorzugt ist das Seilverkürzungsmittel an der Vorderseite der Oberschenkelschiene angeordnet, um eine einfache Bedienung durch den Patienten zu ermöglichen. So kann das Seilverkürzungsmittel etwa eine um eine Drehachse drehbare Scheibe sein, wobei beim Drehen der Scheibe der Seilring um die Drehachse aufgerollt, und dabei das Zugmittel verkürzt wird. Auf diese Weise läßt sich die Vorrichtung zur Stabilisierung eines Gelenks optimal an beliebige Patienten anpassen und es kann über das Seilverkürzungsmittel eine bestimmte Vorspannung der Vorrichtung, d.h. der Ober- bzw. Unterschenkelschiene gegeneinander vorgesehen werden, so daß die Stabilisierungsfunktion beispielsweise auch auf das Gewicht eines Patienten anpassbar ist. Über den Seilzug läßt sich dadurch eine Anpassung der Vorrichtung an verschiedene Gelenkgrößen vorsehen, was eine besonders hohe Flexibilität beim Einsatz der Knierorthese zuläßt.

Bei einer vorteilhaften Weiterbildung der Erfindung werden sowohl der Ober- als auch der Unterschenkel semizirkulär einfaßt. Im Gegensatz zu den bekannten Knieorthesen, die die Ober- bzw. Unterschenkelmuskulatur vollständig umfassen und bei denen es notwendig ist, den Ober- bzw. Unterschenkel fest einzuspannen, um eine Stabilisierung zu ermöglichen, wird bei der erfindungsgemäßen Vorrichtung die Muskulatur nicht mehr abgeschnürt. Durch das erfindungsgemäße Zugmittel wird die Oberschenkelschiene nach vorne und zum Kniegelenk hin gespannt und die Unterschenkelschiene wird nach hinten und nach oben zum Kniegelenk gespannt. Infolgedessen ist die erste Fixierschiene, insbesondere die Unterschenkelschiene, lediglich an der Vorderseite des Unterschenkels geschlossen, während die hintere Seite des Unterschenkels freiliegend sein kann. Die Unterschenkelschiene ist bevorzugt an den Unterschenkel angeformt, mit einer Polsterung versehen und kann zur besseren Stützfunktion auch an den Seiten des Unterschenkels nach hinten geführt sein. Da jedoch keine zirkuläre Einfassung des Unterschenkels notwendig ist, wird sowohl die Durchblutung als auch die Muskulatur durch um den Unterschenkel ringförmig verlaufende Kunststoffbänder mit Klettverschluß nicht abgeschnürt.

Ebenso kann die zweite Fixierschiene, insbesondere die Oberschenkelschiene, an der Vorderseite offen sein. Die Oberschenkelschiene weist erfindungsgemäß eine hintere Stützwand und zwei seitliche Stützwände auf, die miteinander verbindbar sind. Bevorzugt sind an den seitlichen Stützwänden im hinteren Bereich zwei Umlenklager vorgesehen. Im vorderen Bereich der seitlichen Stützflächen sind rechts und links jeweils eine Lagerung für das nach vorne verlaufende Zugmittel vorgesehen, das über dem freiliegenden Oberschenkel vorne an der offenen Seite der Oberschenkelschiene verläuft und dort bevorzugt ein Seilverkürzungsmittel aufweist. Natürlich kann an der offenen Seite der Oberschenkelschiene eine Polsterung zum Schutz des freiliegenden Oberschenkels gegenüber dem Seilverkürzungsmittel vorgesehen sein.

Die Umlenkung des Zugmittels an den zwei seitlichen Umlenklagern bewirkt außerdem, daß bei einer Auslenkung der ersten Fixierschiene, beispielsweise nach vorn, die beiden seitlichen Stützwände der zweiten Fixierschiene, insbesondere der Oberschenkelschiene, zueinander nach innen bewegt werden. Dabei erzeugt die Lagerung des Zugmittels an der Vorderseite der seitlichen Stützwände Kräfte, wodurch die Stützwände seitlich an den Oberschenkeln stärker angepreßt werden.

Bevorzugt sind die Fixierschienen, insbesondere aber die Oberschenkelschiene, in Bezug auf ihren Umfang verstellbar. Beispielsweise kann die Oberschenkelschiene aus drei separaten Schienenwänden, nämlich einer Rückwand und zwei Seitenwänden, beistehen. Die Rückwand kann einen Verstellmechanismus, beispielsweise eine Nut-Feder-Verbindung aufweisen, über die die Seitenwände mit unterschiedlichen Distanzen zueinander an der Rückwand befestigbar sind. Dadurch läßt sich die Oberschenkelschiene bevorzugt auf unterschiedliche Oberschenkelumfänge einstellen.

Die beiden Seitenflächen sind dabei bevorzugt der Form des Oberschenkelknochens im Bereich des Kniegelenks nachgebildet. Beispielsweise kann sich die Oberschenkelschiene von oben nach unten verjüngen, wodurch ein Abrutschen der Oberschenkelschiene, insbesondere der Seitenwände, über das Kniegelenk verhindert wird.

Durch ein elastisches Zugmittel kann eine gewisse Bewegungsfreiheit im zu stabilisierenden Kniegelenk vorgesehen werden. Das kann sich vorteilhaft auf das Gangbild mit der angelegten Knieorthese auswirken.

Eine Fixierschiene, insbesondere eine Unterschenkelschiene, kann eine Halteeinrichtung aufweisen, die den zum Gelenk hin wirkenden Zugkräften des Zugmittels entgegenwirkt. Diese Halteeinrichtung ist bevorzugt ein Fersenumfassungsgurt. Über die Halteeinrichtung läßt sich die Unterschenkelschiene zusätzlich hin zum Fuß spannen, wodurch die Zugkräfte der Vorrichtung zum Knie noch besser ausgleichbar sind. Die Zugkräfte der erfindungsgemäßen Vorrichtung vergrößern sich entsprechend der Abwinkelung des Oberschenkels gegenüber der Vertikalen und stabilisieren dadurch das Kniegelenk bei vergrößerter Abwinkelung des Knies zusätzlich. Insbesondere wenn mit der erfindunsgemäßen Vorrichtung in der Rehabilitationsphase Sport getrieben werden soll, kann die stabilisierende Wirkung am Kniegelenk durch den Fersenumfassungsgurt und ein Vergrößern der Zugkräfte am Zugmittel zusätzlich erhöht werden.

Gemäß einer vorteilhaften Ausführungsform ist ein Bewegungsmechanismus vorgesehen, der ein Führungsmittel zum kurvenförmigen Verschieben des Lagerpunktes einer Fixierschiene aufweist. Die beiden Fixierschienen können somit auch über ein Drehlager verbunden sein, das einerseits ein Verschwenken der beiden Fixierschienen und andererseits ein Verlagern des Drehpunktes entlang einer vorgegebenen Kurve zulässt. Die Verschiebekurve des Drehpunktes kann entsprechend an die natürlichen Gelenkbewegungen angepasst sein und auch ein Spannmittel, beispielsweise eine Feder, aufweisen, das die Unterschenkelschiene bevorzugt zum Gelenk und nach hinten spannt.

Die erfindungsgemäße Verlagerung des Drehpunktes berücksichtigt, dass die Abknickbewegung des Kniegelenks nicht einfach durch ein Scharnier mit festem Drehpunkt nachbildbar ist. Der Drehpunkt zwischen den beiden Fixierschienen verlagert sich erfindungsgemäß entlang der nach anatomischem Vorbild ausgebildeten Verschiebekurve. Erfindungsgemäß ist auch vorgesehen, dass der Bewegungsmechanismus zum kurvenförmigen Verschieben des Drehpunktes mit dem Bewegungsmechanismus mit dem Zugmittel kombinierbar ist. Auf diese Weise entsteht eine besonders vorteilhafte Vorrichtung zum Stabilisieren eines Gelenks.

Bevorzugt weist das kurvenförmige Führungsmittel eine Führungsnut, Führungschiene oder ein spezielles Drehlager auf, das die genannten Ausgleichsbewegung der beiden Fixierschienen ermöglicht. Im einfachsten Fall weist eine Fixierschiene, insbesondere die Unterschenkelschiene, zwei seitlich angeordnete Bolzen auf, die jeweils in am Bewegungsmechanismus oder an der Oberschenkelschiene seitlich angeordnete kurvenförmige Führungsnuten eingreifen. Die Bolzen mit der daran befestigten Unterschenkelschiene lassen sich deswegen entlang der kurvenförmigen Führungsnut verlagern, so daß der Drehpunkt zwischen den Fixierschienen, der durch die Bolzen gebildet ist, kurvenförmig verlagerbar ist.

Weitere vorteilhafte Ausführungsformen der Erfindung ergeben sich aus den Unteransprüchen und der Beschreibung. Mehrere Ausführungsbeispiele der Erfindung sind in der Zeichnung dargestellt und werden nachfolgend beschrieben. Es zeigen, jeweils in schematischer Darstellung,

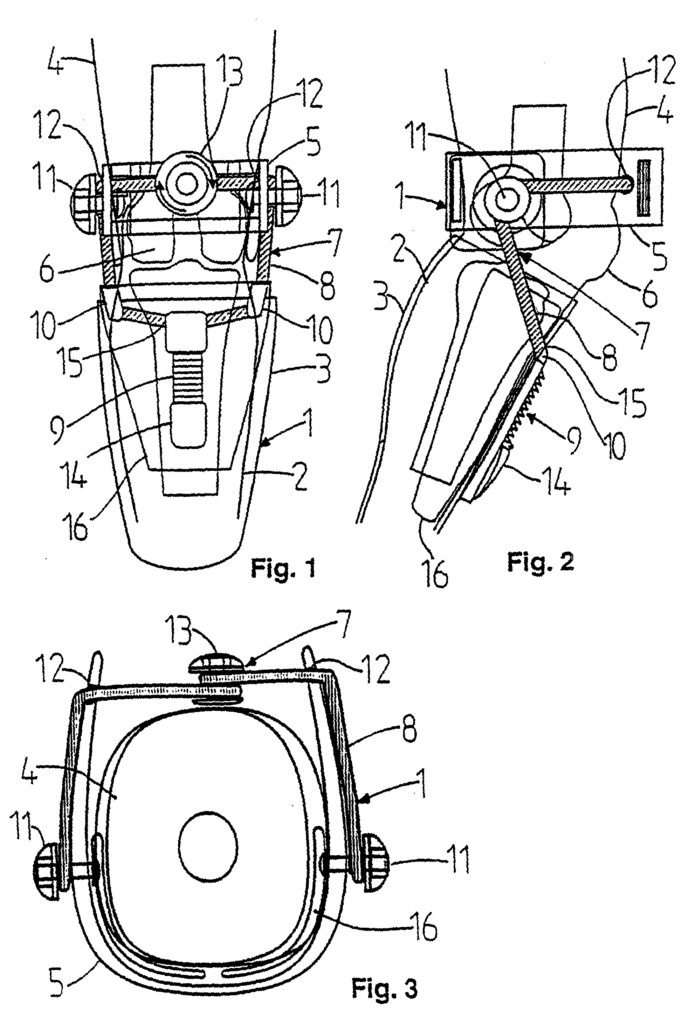

- Figur 1: eine Vorderansicht der erfindungsgemäßen Vorrichtung nach einem ersten Ausführungsbeispiel,
- Figur 2: eine Seitenansicht der erfindungsgemäßen Vorrichtung nach dem ersten Ausführungsbeispiel,
- Figur 3: eine Draufsicht auf die erfindungsgemäße Vorrichtung nach dem ersten Ausführungsbeispiel,
- Figur 4: eine Seitenansicht eines Teils der erfindungsgemäßen Vorrichtung nach einem zweiten Ausführungsbeispiel,
- Figur 5: eine Vorderansicht des Teils der erfindungsgemäßen Vorrichtung von Figur 4,
- Figur 6: eine Seitenansicht einer am Bein angelegten erfindungsgemäßen Vorrichtung nach dem zweiten Ausführungsbeispiel und
- Figur 7: eine Vorderansicht der am Bein angelegten Vorrichtung nach dem zweiten Ausführungsbeispiel.

Die erfindungsgemäße Knieorthese 1 weist eine an einem Unterschenkel 2 festlegbare Unterschenkelschiene 3, eine an einem Oberschenkel 4 festlegbare Oberschenkelschiene 5 und einen das Kniegelenk 6 überbrückenden Bewegungsmechanismus 7 auf. Die Unterschenkelschiene 3 ist unterhalb des Kniegelenks 6 und die Oberschenkelschiene 5 oberhalb des Kniegelenks 6 anordenbaren und beide sind über den Bewegungsmechanismus 7 miteinander verbunden.

Der Bewegungsmechanismus 7 weist einen Seilzug 8 auf, der einen geschlossenen Ring ausbildet. Der Seilzug 8 ist in einem oberen vorderen Bereich der Unterschenkelschiene 3 an einem Verstellelement 9 eingehängt. Der Seilzug 8 ist zusätzlich über zwei an der Unterschenkelschiene 3 angeordnete Fixierelemente 10 verschiebbar gelagert und verläuft zu beiden Seiten des Kniegelenks jeweils zu einem an der Oberschenkelschiene 5 seitlich und hinten angeordneten Umlenklager 11. Der Seilzug 8 verläuft an den Seiten des Kniegelenks 6 von der Vorderseite des Unterschenkels 2 zur Rückseite des Oberschenkels 4. Auf diese Weise bildet die erfindungsgemäße Knieorthese 1 die natürlichen Zugkräfte des menschlichen Kreuzbandes im Bereich des Kniegelenks 6, jedoch außerhalb des Beines, nach.

Von den beiden Umlenklagern 11 verläuft der Seilzug 8 entlang den Seitenwänden der Oberschenkelschiene 5 nach vorne und wird über weitere Führungselemente 12, beispielsweise Bohrungen an den Seitenwänden, an der Vorderseite der Oberschenkelschiene 5 zu einem Seilverkürzungsmittel 13 geführt.

Das Seilverkürzungsmittel 13 ist eine drehbare Scheibe, bei deren Betätigung die Länge des Seilzugs 8 verkürzt bzw. verlängert werden kann. Auf diese Weise kann die durch den Seilzug 8 erzeugte Spannung zwischen der Oberschenkelschiene 5 und der Unterschenkelschiene 3 veränderbar sein. Die Seitenwände der Oberschenkelschiene 5 können mit einer auf die Form des Oberschenkelknochens angepaßten Polsterung, wie etwa einem Luftkissenpolster, versehen sein.

Das Verstellelement 9 bewirkt eine Verlagerung des unteren Befestigungspunktes des Seilzugs 8 an der Unterschenkelschiene 3. Durch Betätigen des Verstellelements kann der Befestigungspunkt entlang der Vorderseite der Unterschenkelschiene 3 in einer Richtung parallel zum Unterschenkelverlauf verlagerbar sein. Das Verstellelement 9 ist in der Art einer Skischuhschnalle 14 ausgebildet und kann den unteren Befestigungspunkt 15 des Seilzugs 8 um eine vorgegebene Distanz verlagern. Dadurch wird der Seilzug 8 nach unten gespannt. Außerdem kann vorgesehen sein, daß zum Ablegen der Knieorthese 1 der Seilzug 8 am unteren Befestigungspunkt 15 aushängbar ist. Alternativ kann vorgesehen sein, daß der Seilzug 8 im Bereich des Seilverkürzungsmittels 13 über einen Rastverschluß auftrennbar ist.

Der erfindungsgemäße Verlauf des Seilzugs 8 zwischen der Vorderseite der Unterschenkelschiene 3 und der Rückseite der Oberschenkelschiene 5 erzeugt eine Stabilisierung des Kniegelenks 6 durch Nachhintenziehen der Unterschenkelschiene 3 und Nachvorneziehen der Oberschenkelschiene 5. Der Seilzug 8 könnte deswegen auch an der Rückseite der Oberschenkelschiene 5 ohne Umlenkung an den Umlenklagern 11 geschlossen sein.

Die Unterschenkelschiene 3 weist eine an den Unterschenkel 2 angeformte Schiene mit einer innenliegenden Polsterung auf. Die Unterschenkelschiene 3 ist an der Rückseite des Unterschenkels 2 nicht geschlossen, weist also im wesentlichen einen halbkreisförmigen Querschnitt auf. Die Oberschenkelschiene 5 weist ebenfalls einen halbkreisförmigen Querschnitt auf und ist an der Rückseite des Oberschenkels 4 geschlossen und an der Vorderseite des Oberschenkels 4 offen. Auch hier verbessert eine Polsterung 16 den Tragkomfort der Oberschenkelschiene 5. Die Unterschenkelschiene 3 ist eine in Richtung längs zum Unterschenkel 2 langgestreckte Schiene, während die Oberschenkelschiene 5 ein schmales, den Oberschenkel 4 umgreifendes halbkreisförmiges starres Bandsegment aus Kunststoff bildet. Die Oberschenkelschiene 5 kann aus einer separaten Rückwand und daran verstellbar einhängbaren Seitenwänden bestehen.

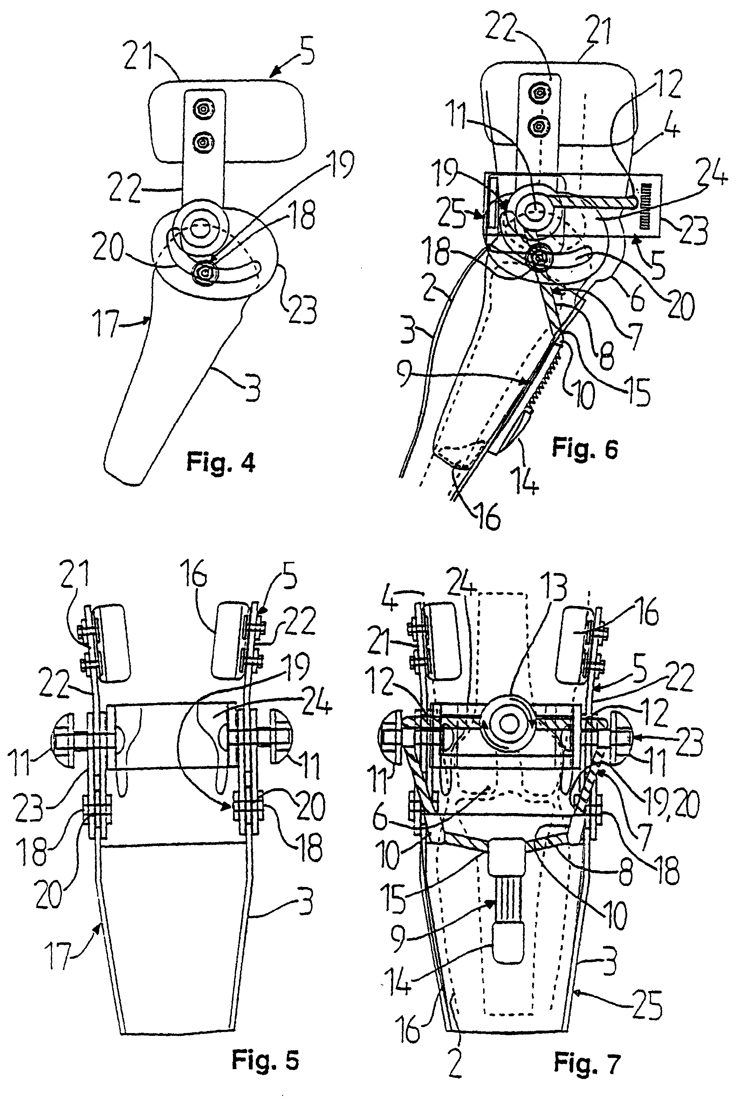

In den Figuren 4 und 5 ist ein Teil einer zweiten Ausführungsform der vorliegenden Erfindung dargestellt. Hierbei sind gleiche oder ähnliche Elemente wie bei der ersten Ausführungsform durch dieselben Bezugszeichen dargestellt. Die Knieorthese 17 weist eine Unterschenkelschiene 3 mit zwei seitlich angeordneten Lagerstiften 18 auf, die entlang einer im Bewegungsmechanismus 19 angeordneten kurvenförmigen Führungsnut 20 verlagerbar sind. Die seitlich am Bewegungsmechanismus 19 und gegenüber den Seitenwänden der Unterschenkelschiene 3 angeordneten Führungsnuten 20 bilden zusammen mit den Lagerstiften 18 ein örtlich verschiebbares Drehlager zwischen dem Bewegungsmechanismus 19 und der Unterschenkelschiene 3. Die Oberschenkelschiene 5 ist zweiteilig ausgebildet, mit einem oberen Teil 21 mit einer innenliegenden Polsterung 16, der über zwei seitlich angeordneten Stege 22 mit einem unteren Teil 23 der Oberschenkelschiene verbunden ist.

Der untere Teil 23 der Oberschenkelschiene 5 weist einen in seiner Form optimierten Aufnahmebereich 24 für einen unteren Abschnitt des Oberschenkels 4 des Patienten auf. Zur Kombination mit einer Knieorthese 1 mit einem Seilzug 8 weist die Knieorthese 17 vorbereitete seitliche Umlenklager 11 auf. Der in den Figuren 4 und 5 dargestellte Teil der erfindungsgemäßen zweiten Ausführungsform dient zur Stabilisierung des Kniegelenks. Es kann dabei noch ein elastisches Element, beispielsweise eine Spiralfeder vorgesehen sein, das die Lagerstifte 18 bevorzugt in einer Ruhelage im hinteren oder vorderen Bereich der Führungsnut festhält.

Wie aus den Figuren 6 und 7 ersichtlich, besteht die zweite Ausführungsform der erfindungsgemäßen Knieorthese 25 im übrigen aus einer Kombination der ersten Ausführungsform mit der in den Figuren 4 und 5 dargestellten Vorrichtung. Dadurch entsteht eine hervorragend stabilisierende Knieorthese, die zusätzlich entlang anatomisch sinnvoller Bewegungskurven entlang der kurvenförmigen Führungsnut 20 einen verlagerbaren Drehpunkt aufweist. An der Unterschenkelschiene 3 kann noch ein nicht dargestellter Fersenumfassungsgurt vorgesehen sein, der die Unterschenkelschiene 3 in ihrer Position festhält und gegen Verschieben in Richtung des Kniegelenks sichert.

## Patentansprüche

1. Vorrichtung zur Stabilisierung eines ein erstes Körperglied mit einem zweiten Körperglied verbindendenden Gelenks, insbesondere Kniegelenks,
mit einer am ersten Körperglied festlegbaren ersten Fixierschiene (3), die an der Vorderseite des ersten Körperglieds anlegbar ist,
einer am zweiten Körperglied festlegbaren zweiten Fixierschiene (5), welche an der Rückseite des zweiten Körperglieds anlegbar ist,
und einem Zugmittel (8), das an der Vorderseite der ersten Fixierschiene (3) beidseitig am Gelenk vorbei zur zweiten Fixierschiene (5) geführt werden kann, wobei, wenn die Vorrichtung am Gelenk angebracht ist, das Zugmittel (8) von der Vorderseite der ersten Fixierschiene (3) zu einem hinteren Bereich der zweiten Fixierschiene (5) verläuft, **dadurch gekennzeichnet, dass** das Zugmittel auf die erste Fixierschiene (3) eine zum Gelenk und nach hinten gerichtete Zugkraft und auf die zweite Fixierschiene (5) eine zum Gelenk und nach vorne gerichtete Zugkraft ausübt, so dass das erste Körperglied zum Gelenk und nach hinten und das zweite Körperglied zum Gelenk und nach vorne gespannt wird.

2. Vorrichtung nach Anspruch 1, **dadurch gekennzeichnet, dass** das Zugmittel (8) ein Seilzug oder ein flexibler Kunststoff- oder Drahtstab ist.

3. Vorrichtung nach einem der Ansprüche 1 oder 2, **dadurch gekennzeichnet, dass** das Zugmittel (8)von der Vorderseite der ersten Fixierschiene (3) zu einem hinteren Bereich der Seitenfläche oder zur Rückseite der zweiten Fixierschiene (5) verläuft.

4. Vorrichtung nach einem der Ansprüche 1 bis 3, **dadurch gekennzeichnet, dass** das Zugmittel (8) an der Seitenfläche der zweiten Fixierschiene (5) über ein Umlenklager (11) umlenkbar ist und von dort zur Vorderseite der zweiten Fixierschiene (5) verläuft.

5. Vorrichtung nach einem der Ansprüche 1 bis 4, **dadurch gekennzeichnet, dass** das Zugmittel (8) als Seilring ausgebildet ist.

6. Vorrichtung nach einem der Ansprüche 1 bis 5, **dadurch gekennzeichnet, dass** ein erstes Verstellelement (9) zum Verlagern des Befestigungs- oder Umlenkpunktes des Zugmittels an der Vorderseite der ersten Fixierschiene vorgesehen ist.

7. Vorrichtung nach einem der Ansprüche 1 bis 6, **dadurch gekennzeichnet, dass** durch ein Seilverkürzungsmittel (13) die Länge des Zugmittels (8) einstellbar ist.

8. Vorrichtung nach Anspruch 7, **dadurch gekennzeichnet, dass** das Seilverkürzungsmittel (13) an der Vorderseite der zweiten Fixierschiene (5) angeordnet ist.

9. Vorrichtung nach einem der Ansprüche 1 bis 8, **dadurch gekennzeichnet, dass** bei am gestreckten Gelenk festgelegter Vorrichtung die erste Fixierschiene (3) an der Vorderseite des Gelenks geschlossen ist.

10. Vorrichtung nach einem der Ansprüche 1 bis 9, **dadurch gekennzeichnet, dass** die zweite Fixierschiene (5) an der Vorderseite offen ist.

11. Vorrichtung nach einem der Ansprüche 1 bis 10, **dadurch gekennzeichnet, dass** die zweite Fixierschiene (5) in Bezug auf ihren Umfang verstellbar ist.

12. Vorrichtung nach einem der Ansprüche 1 bis 11, **dadurch gekennzeichnet, dass** das Zugmittel (8) elastisch ist.

13. Vorrichtung nach einem der Ansprüche 1 bis 12, **dadurch gekennzeichnet, dass** eine Fixierschiene eine Halteeinrichtung aufweist, die den zum Gelenk hin wirkenden Zugkräften des Zugmittels (8) entgegenwirkt.

14. Vorrichtung nach Anspruch 13, **dadurch gekennzeichnet, dass** die Halteeinrichtung einen Fersenumfassungsgurt aufweist.

15. Vorrichtung nach einem der Ansprüche 1 bis 14 **gekennzeichnet durch** einen Bewegungsmechanismus (19), der ein Führungsmittel zum kurvenförmigen Verschieben des Lagerpunktes einer Fixierschiene aufweist.

16. Vorrichtung nach Anspruch 15, **dadurch gekennzeichnet, dass** das kurvenförmige Führungsmittel eine Führungsnut (20), Führungschiene oder ein spezielles Drehlager aufweist.

17. Vorrichtung nach Anspruch 15 oder 16, **dadurch gekennzeichnet, dass** eine Fixierschiene zwei seitlich angeordnete Bolzen (18) aufweist, die jeweils in eine am Bewegungsmechanismus (19) seitlich angeordnete kurvenförmige Führungsnut (20) eingreifen.

## Claims

1. Device for stabilizing a joint, in particular a knee joint, connecting a first body part to a second body part, with a first fixation splint (3) which can be secured on the first body part and can be placed on the anterior face of the first body part, with a second fixation splint (5) which can be secured on the second body part and can be placed on the posterior face of the second body part, and with a tensioning means (8) which on the anterior face of the first fixation splint (3) can be guided along both sides of the joint to the second fixation splint (5), and, when the device is arranged on the joint, the tensioning means (8) extends from the anterior face of the first fixation splint (3) to a rear area of the second fixation splint (5), **characterized in that** the tensioning means exerts on the first fixation splint (3) a tensile force which is directed towards the joint and rearwards and exerts on the second fixation splint (5) a tensile force which is directed towards the joint and forwards, so that the first body part is tensioned towards the joint and rearwards and the second body part is tensioned towards the joint and forwards.

2. Device according to Claim 1, **characterized in that** the tensioning means (8) is a cable or a flexible plastic rod or wire rod.

3. Device according to either of Claims 1 and 2, **characterized in that** the tensioning means (8) extends from the anterior face of the first fixation splint (3) to a rear area of the side face or to the posterior face of the second fixation splint (5).

4. Device according to one of Claims 1 to 3, **characterized in that** the tensioning means (8) on the side face of the second fixation splint (5) can be deflected via a deflection bearing (11) and from there extends to the anterior face of the second fixation splint (5).

5. Device according to one of Claims 1 to 4, **characterized in that** the tensioning means (8) is designed as a cable ring.

6. Device according to one of Claims 1 to 5, **characterized in that** a first adjustment element (9) for shifting the securing or deflection point of the tensioning means is provided on the anterior face of the first fixation splint.

7. Device according to one of Claims 1 to 6, **characterized in that** the length of the tensioning means (8) can be adjusted by a cable-shortening means (13).

8. Device according to Claim 7, **characterized in that** the cable-shortening means (13) is arranged on the anterior face of the second fixation splint (5).

9. Device according to one of Claims 1 to 8, **characterized in that**, with the device secured on the extended joint, the first fixation splint (3) is closed on the anterior face of the joint.

10. Device according to one of Claims 1 to 9, **characterized in that** the second fixation splint (5) is open on the anterior face.

11. Device according to one of Claims 1 to 10, **characterized in that** the second fixation splint (5) is adjustable in terms of its circumference.

12. Device according to one of Claims 1 to 11, **characterized in that** the tensioning means (8) is elastic.

13. Device according to one of Claims 1 to 12, **characterized in that** one fixation splint has a holding means which counteracts the tensile forces of the tensioning means (8) which act towards the joint.

14. Device according to Claim 13, **characterized in that** the holding means has a heel strap.

15. Device according to one of Claims 1 to 14, **characterized by** a movement mechanism (19) which has a guide means for a curve-shaped displacement of the bearing point of one fixation splint.

16. Device according to Claim 15, **characterized in that** the curve-shaped guide means has a guide groove (20), guide rail or a special pivot bearing.

17. Device according to Claim 15 or 16, **characterized in that** one fixation splint has two laterally arranged bolts (18) which each engage in a curve-shaped guide groove (20) arranged laterally on the movement mechanism (19).

## Revendications

1. Dispositif pour stabiliser une articulation reliant un premier membre du corps avec un deuxième membre du corps, en particulier une articulation du genou, comportant
une première attelle de fixation (3), susceptible d'être fixée sur le premier membre du corps et qui peut être mise en appui sur la face antérieure du premier membre du corps,
une deuxième attelle de fixation (5), susceptible d'être fixée sur le deuxième membre du corps et qui peut être mise en appui sur la face postérieure du deuxième membre du corps,
et un moyen de traction (8) qui peut être guidé sur la face antérieure de la première attelle de fixation (3), des deux côtés le long de l'articulation en direction de la deuxième attelle de fixation (5), et lorsque le dispositif est monté sur l'articulation, le moyen de traction (8) s'étend depuis la face antérieure de la première attelle de fixation (3) en direction d'une région postérieure de la deuxième attelle de fixation (5), **caractérisé en ce que** le moyen de traction exerce sur la première attelle de fixation (3) une force de traction dirigée vers l'articulation et vers l'arrière et exerce sur la deuxième attelle de fixation (5) une force de traction dirigée vers l'articulation et vers l'avant, de sorte que le premier membre du corps est tendu vers l'articulation et vers l'arrière et que le deuxième membre du corps est tendu vers l'articulation et vers l'avant.

2. Dispositif selon la revendication 1, **caractérisé en ce que** le moyen de traction (8) est un câble gainé ou une tige en matière plastique ou en fil métallique flexible.

3. Dispositif selon l'une ou l'autre des revendications 1 et 2, **caractérisé en ce que** le moyen de traction (8) s'étend depuis la face antérieure de la première attelle de fixation (3) vers une région postérieure de la surface latérale ou vers la face postérieure de la deuxième attelle de fixation (5).

4. Dispositif selon l'une des revendications 1 à 3, **caractérisé en ce que** le moyen de traction (8) peut être dévié sur la surface latérale de la deuxième attelle de fixation (5) via un palier de déviation (11) et depuis là vers la face antérieure de la deuxième attelle de fixation (5).

5. Dispositif selon l'une des revendications 1 à 4, **caractérisé en ce que** le moyen de traction (8) est réalisé sous forme de câble en forme d'anneau.

6. Dispositif selon l'une des revendications 1 à 5, **caractérisé en ce qu'**un premier élément de réglage (9) pour déplacer le point de fixation ou le point de déviation du moyen de traction est prévu sur la face antérieure de la première attelle de fixation.

7. Dispositif selon l'une des revendications 1 à 6, **caractérisé en ce qu'**on peut régler la longueur du moyen de traction (8) par un moyen de raccourcissement de câble (13).

8. Dispositif selon la revendication 7, **caractérisé en ce que** le moyen de raccourcissement de câble (13) est agencé sur la face antérieure de la deuxième attelle de fixation (5).

9. Dispositif selon l'une des revendications 1 à 8, **caractérisé en ce que** dans le cas où le dispositif est fixé sur l'articulation en extension, la première attelle de fixation (3) est fermée sur la face antérieure de l'articulation.

10. Dispositif selon l'une des revendications 1 à 9, **caractérisé en ce que** la deuxième attelle de fixation (5) est ouverte sur la face antérieure.

11. Dispositif selon l'une des revendications 1 à 10, **caractérisé en ce que** la deuxième attelle de fixation (5) est réglable par rapport à sa circonférence.

12. Dispositif selon l'une des revendications 1 à 11, **caractérisé en ce que** le moyen de traction (8) est élastique.

13. Dispositif selon l'une des revendications 1 à 12, **caractérisé en ce qu'**une attelle de fixation présente un système de retenue qui s'oppose aux forces du moyen de traction (8) qui agissent vers l'articulation.

14. Dispositif selon la revendication 13, **caractérisé en ce que** le dispositif de retenue présente une sangle de saisie du talon.

15. Dispositif selon l'une des revendications 1 à 14, **caractérisé par** un mécanisme de mouvement (19) qui présente un moyen de guidage pour déplacer sur une courbe le point d'appui d'une attelle de fixation.

16. Dispositif selon la revendication 15, **caractérisé en ce que** le moyen de guidage en forme de courbe présente une rainure de guidage (20), un . rail de guidage ou un palier rotatif spécial.

17. Dispositif selon l'une ou l'autre des revendications 15 et 16, **caractérisé en ce qu'**une attelle de fixation présente deux goujons (18) agencés latéralement qui s'engagent chacun dans une rainure de guidage (20) en forme de courbe agencée latéralement sur le mécanisme de mouvement (19).
